# EUROPÄISCHE PATENTANMELDUNG

(11) **EP 2 881 088 A1**
(43) Veröffentlichungstag der Anmeldung: **10.06.2015**
(21) Anmeldenummer: 14002848.1
(22) Anmeldetag: 14.08.2014
(51) Int. Cl.: A61F 2/91, A61B 19/00

(54) **Körperimplantat mit einem Markerelement**

(30) Priorität: 04.12.2013 DE 102013020689
(71) Anmelder: Admedes Schuessler GmbH, 75179 Pforzheim (DE)
(72) Erfinder: Knobloch, Thomas, 75179 Pforzheim (DE); Steiner, Ralf, 75179 Pforzheim (DE)
(74) Vertreter: Müller-Boré & Partner Patentanwälte PartG mbB

(57) **Zusammenfassung**

Die Erfindung betrifft ein Körperimplantat 1, insbesondere einen Stent, zum Einführen oder Implantieren in einen lebenden Körper mit einem Markerelement 20 aus einem röntgensichtbaren Material, das in einen Ausschnitt 10 des Körperimplantats 1 eingesetzt ist, wobei der Ausschnitt 10 zwei Paare Flächen aufweist, so dass ein Paar erster Flächen 11 eine erste Hinterschneidung in einer Richtung zu dem Inneren des Körperimplantats 1 aufweist, während das andere Paar zweiter Flächen 12 eine zweite Hinterschneidung in der im wesentlichen entgegengesetzten Richtung aufweist.

## Beschreibung

Die vorliegende Erfindung betrifft ein Körperimplantat, insbesondere einen Stent zum Einführen in einen lebenden Körper mit einer guten Röntgensichtbarkeit sowie auf ein Verfahren zum Herstellen eines derartigen Körperimplantats und auf ein Verfahren zum Ermöglichen bzw. Verbessern der Röntgensichtbarkeit eines Körperimplantats.

Körperimplantate bzw. Stents dieser Art schützen Kanäle lebender Körper wie beispielsweise Blutgefäße, Speiseröhre, Harnröhre oder Nierengänge durch Einführen des Stents und Expandieren des Stents im Inneren des Körperkanals. Auf diese Weise kann ein Kollabieren oder Verschließen des jeweiligen Körperkanals verhindert werden. Darüber hinaus wird ein Stent beispielsweise für intercerebrale Gefäßaussackungen, sogenannte Aneurysmen eingesetzt, die die häufigste Ursache für nicht-traumatische Subarachnoidal-Blutungen sind. Ihre Inzidenz liegt in der Gesamtbevölkerung bei 1%, nach Autopsiestudien sogar bis zu 9%. Patomorphologisch sind intercerebrale Aneurysmen in der Regel echte, sacurale Aneurysmen, die meist in Gefäßaufzweigungen lokalisiert sind (vgl. beispielsweise Schumacher M. "Diagnostic work-up in cerebral aneurysms" in Nakstadt PHj (ed): "cerebral aneurysms", pp 13-24, Bologna: Centauro (2000)).

Darüber hinaus können derartige Körperimplantate bzw. Stents als Träger von Medikamenten dienen, um eine lokale Therapie innerhalb des Körperkanals zu ermöglichen.

Diese Stents werden in einem kollabierten Zustand in einen Körperkanal eingeführt und nach Anordnung des Stents in dem Körperkanal expandiert. Diese Stents bestehen üblicherweise aus rostfreiem Edelstahl oder einer Kobalt-Chrom-Tantal-Legierung. Die Stents werden bevorzugt durch eine Aufweitungseinrichtung wie beispielsweise einem Ballonkatheter in den Körperkanal eingeführt und dort aufgeweitet.

Die Stents können jedoch auch aus anderen Werkstoffen wie beispielsweise Polymeren, selbstabbaubaren Werkstoffen wie beispielsweise Milchsäure-Werkstoffen bzw. Derivaten sowie aus Nitinol (Nickel-Titan-Legierungen) und/oder aus anderen selbstexpandierbaren Werkstoffen wie beispielsweise sogenannten Formgedächtnis-Werkstoffen bestehen.

Um die Röntgensichtbarkeit von diesen Stents zu erhöhen, werden diese vielfach mit zusätzlichen Elementen (sogenannten Markern) versehen, die aus einem Material mit hoher Röntgensichtbarkeit hergestellt sind.

Die Aufgabe der Erfindung besteht in der Schaffung eines Körperimplantats mit einer guten Röntgensichtbarkeit, das einfach und kostengünstig hergestellt werden kann, sowie eines Verfahrens zum Herstellen eines derartigen Körperimplantats und eines Verfahrens zum Ermöglichen bzw. Verbessern der Röntgensichtbarkeit eines Körperimplantats.

Diese Aufgabe wird durch ein die Merkmale der unabhängigen Ansprüche gelöst. Vorteilhafte Weiterbildungen der Erfindung sind in den abhängigen Ansprüchen definiert.

Gemäß einem ersten Gesichtspunkt wird ein Körperimplantat, insbesondere ein Stent, zum Einführen oder Implantieren in einen lebenden Körper mit einem Markerelement aus einem röntgensichtbaren Material, das in einen Ausschnitt des Körperimplantats eingesetzt ist, zur Verfügung gestellt. Der Ausschnitt weist zwei Paare Flächen auf, so dass ein Paar erster Flächen eine erste Hinterschneidung in einer Richtung zu dem Inneren des Körperimplantats aufweist, während das andere Paar zweiter Flächen eine zweite Hinterschneidung in der im wesentlichen entgegengesetzten Richtung aufweist.

Aufgrund der beiden entgegengesetzten Hinterschneidungen bzw. Formschlüsse ist das Markerelement in dem Ausschnitt fixiert. Zum Einsetzen weist das Markerelement dabei höchstens einen der beiden Formschlüsse auf, um den Einsetzvorgang nicht zu behindern. Danach wird das Markerelement verformt, um beide Hinterscheidungen aufzuweisen. Diese Hinterschneidungen werden durch die Flächen, die vorzugsweise durch Schneiden des Ausschnitts gebildet werden, erzeugt, so dass es zwei in entgegengesetzte Richtungen zeigende Keile gibt, die das Markerelement in dem Ausschnitt fixieren.

Vorzugsweise weist das Markerelement komplementäre Paare Flächen auf und eines der Paare Flächen bildet einen Keil an dem Markerelement und das andere Paar Flächen bildet einen Keil an dem Körperimplantat bzw. dem Ausschnitt.

Weiter bevorzugt ist der Ausschnitt in einem zumindest teilweise runden oder bogenförmigen Abschnitt des Körperimplantats angeordnet und die Flächen sind in Umfangsrichtung des Körperimplantats versetzt und/oder das Markerelement hat einen Querschnitt in der Form eines Kreisbogens.

Vorzugsweise ist das Markerelement in den Ausschnitt eingepresst oder eingenietet und/oder der Ausschnitt hat im wesentlichen die Form eines Buchstaben A, H, N, V oder Z.

Weiter bevorzugt ist das Markerelement nach dem Einpressen bzw. Einnieten innen und/oder außen im wesentlichen eben mit einer Implantatstruktur des Körperimplantats. Dies wird insbesondere dadurch erzielt, dass das Markerelement vor dem Einpressen eine dem Ausschnitt ähnliche Form hat und eine Materialmenge des Markerelements so bemessen ist, dass es nach dem Verpressen den Ausschnitt im wesentlichen vollständig ausfüllt. Falls gewünscht, kann noch eine Oberflächenbehandlung an dem Markerelement und/oder der daran angrenzenden Implantatstruktur vorgenommen werden, wie beispielsweise Schleifen, Ätzen, Elektropolieren, Beizen, Trommeln etc.

Vorzugsweise weist das Markerelement ein Material mit hoher Absorption für Röntgenstrahlen, wie beispielsweise Gold, Platin, Tantal, Platinlegierung, Platin-Iridium, Niob, auf.

Gemäß einem weiteren Gesichtspunkt wird ein Verfahren zum Verbessern der Röntgensichtbarkeit eines Körperimplantats, insbesondere eines Stents, mit folgenden Schritten geschaffen:
Formen eines Ausschnitts in dem Körperimplantat mittels eines Lasers, so dass der Ausschnitt zwei Paare Flächen aufweist und ein Paar Flächen eine erste Hinterschneidung in einer Richtung zu dem Inneren des Körperimplantats aufweist, während das andere Paar Flächen eine zweite Hinterschneidung in der im wesentlichen entgegengesetzten Richtung aufweist, und
Einsetzen und Fixieren eines Markerelements aus einem Material mit hoher Absorption für Röntgenstrahlen in dem Ausschnitt.

Vorzugsweise hat das Markerelement vor dem Einsetzen in den Ausschnitt eine im wesentlichen komplementäre Form bezüglich der Form des Ausschnitts, weist jedoch höchstens eine der beiden Hinterschneidungen auf. Das Markerelement wird nach dem Einsetzen so verformt, dass es beide Hinterschneidungen aufweist.

Weiter bevorzugt wird der Ausschnitt in einem zumindest teilweise runden oder bogenförmigen Abschnitt des Körperimplantats geformt und die Flächen werden in Umfangsrichtung des Körperimplantats versetzt geformt und/oder das Markerelement wird aus einem Rohr geschnitten, um einen Querschnitt in der Form eines Kreisbogens zu haben.

Gemäß einem weiteren Gesichtspunkt wird ein Verfahren zum Herstellen eines Körperimplantats, insbesondere eines Stents, mit einem röntgensichtbaren Markerelement mit folgenden Schritten geschaffen:
Formen eines Ausschnitts in dem Körperimplantat, so dass der Ausschnitt zwei Paare Flächen aufweist und ein Paar Flächen eine erste Hinterschneidung in einer Richtung zu dem Inneren des Körperimplantats aufweist, während das andere Paar Flächen eine zweite Hinterschneidung in der im wesentlichen entgegengesetzten Richtung aufweist, und
Einpressen bzw. Einnieten eines Markerelements aus einem Material mit hoher Absorption für Röntgenstrahlen.

Nachfolgend wird ein Ausführungsbeispiel der Erfindung unter Bezugnahme auf die beigefügten Zeichnungen näher erläutert.

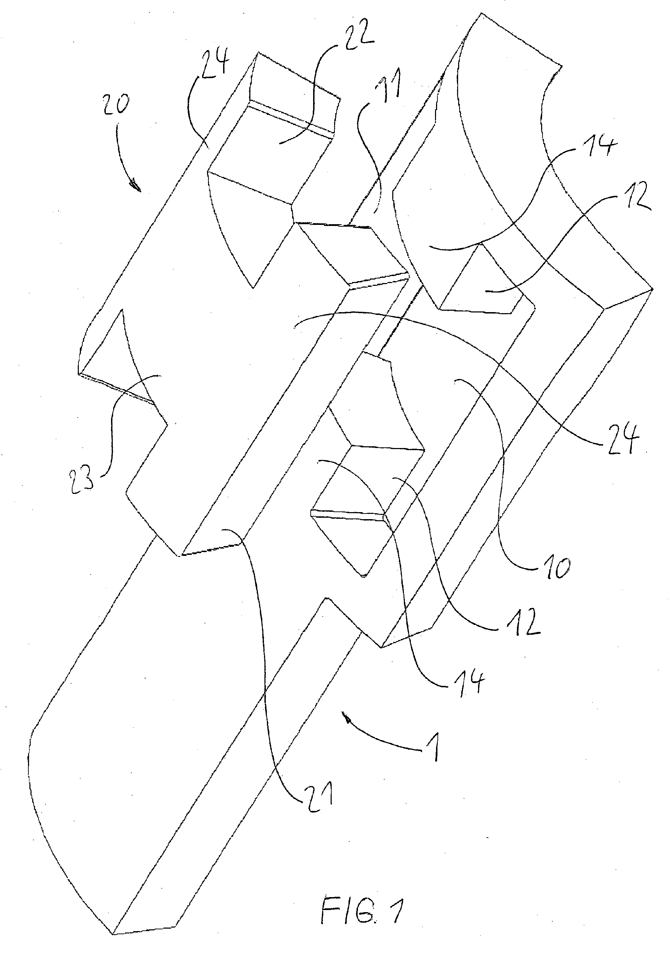

Fig. 1 zeigt in einer perspektivischen Ansicht einen Abschnitt des Körperimplantats gemäß einem ersten Ausführungsbeispiel mit dem in den Ausschnitt des Körperimplantats einzusetzenden Markerelement.

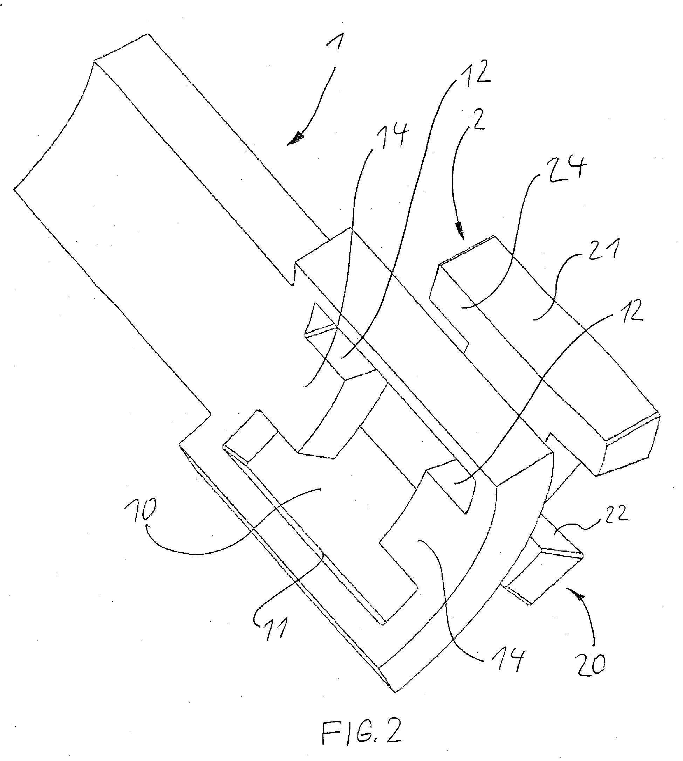

Fig. 2 zeigt ebenfalls eine perspektivische Ansicht des Körperimplantats mit dem Markerelement, jedoch von der anderen Seite als Fig. 1.
Fig. 3 zeigt das Körperimplantat mit dem eingesetzten Markerelement.
Fig. 4 zeigt ebenfalls das Körperimplantat mit dem eingesetzten Markerelement, jedoch von der anderen Seite als Fig. 3.
Fig. 5 zeigt eine Draufsicht auf das Körperimplantat mit dem eingesetzten Markerelement.
Fig. 6 zeigt einen Schnitt durch das Körperimplantat mit dem eingesetzten Markerelement entlang der Linie A-A von Fig. 5.
Fig. 7 zeigt einen Schnitt durch einen geradlinigen Abschnitt des Körperimplantats mit dem eingesetzten Markerelement.
Fig. 8 zeigt eine Abwandlung mit nur einer hinterschnittenen Fläche.
Fig. 9 zeigt ein Körperimplantat gemäß einem zweiten Ausführungsbeispiel ohne das Markerelement, wobei Fig. 9a eine perspektivische Ansicht zeigt und Fig. 9b eine Seitenansicht zeigt.
Fig. 10 zeigt das Körperimplantat von Fig. 9 in einer Draufsicht.

In den Figuren ist ein Abschnitt eines Körperimplantats 1 gezeigt, der beispielsweise eine Querschnittsform in der Gestalt eines Kreisbogens hat. Die Erfindung ist jedoch nicht hierauf beschränkt, sondern der Abschnitt des Körperimplantats 1, in dem sich ein Ausschnitt 10 zum Einsetzen eines Markerelements 20 befindet, kann jede andere Gestalt haben. Bevorzugt hat der Abschnitt des Körperimplantats 1 die Kreisbogenform, wenn das Körperimplantat 1 beispielsweise ein zylindrischer Stent ist. Die Erfindung ist jedoch nicht auf Stents als Körperimplantate 1 beschränkt, sondern kann auf jedes andere Körperimplantat mit jedweder Gestalt angewandt werden.

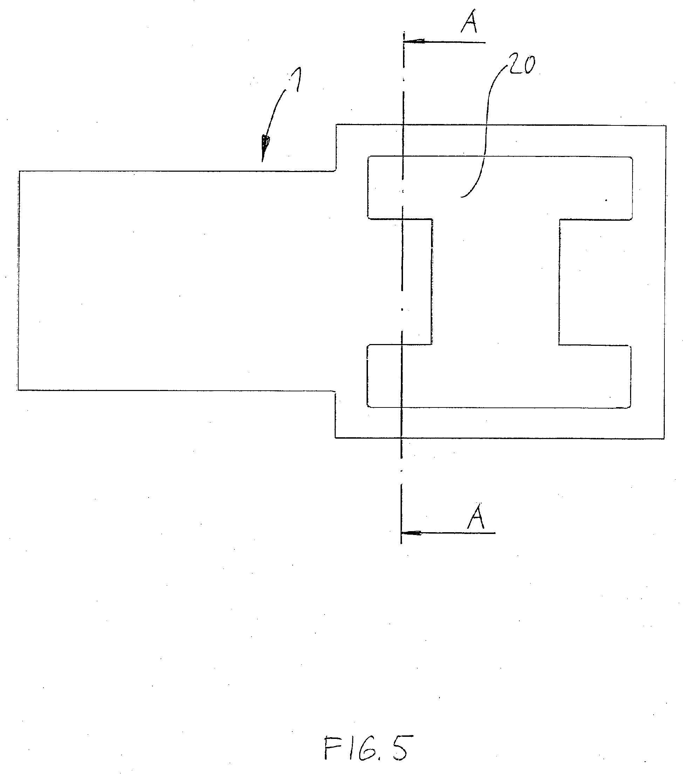

In dem gezeigten Ausführungsbeispiel hat der Ausschnitt 10 in der Draufsicht von Fig. 5 im wesentlichen die Form eines Buchstaben H. Die Erfindung ist jedoch nicht darauf beschränkt, sondern der Ausschnitt 10 kann auch andere Gestalten haben, wie beispielsweise im wesentlichen die Form eines Buchstaben A, N, V oder Z.

Dabei weist der Ausschnitt 10 ein Paar erster Flächen 11 sowie ein Paar zweiter Flächen 12 auf. Diese ersten und zweiten Flächen 11, 12 werden bevorzugt durch Laserschneiden in einem Laserschnitt-Vorgang gebildet. Das in den Ausschnitt 10 zumindest teilweise einzusetzende Markerelement 20 hat eine zu dem Ausschnitt 10 des Körperimplantats 1 im wesentlichen komplementäre Form, in dem gezeigten Ausführungsbeispiel ebenfalls die Form eines Buchstaben H mit einem Paar erster Flächen 21 sowie einem Paar zweiter Flächen 22.

Dabei sind die ersten Flächen 21 derart konfiguriert, um ein Verlagern des Markerelements 20 in einer Richtung zu der Innenseite des Körperimplantats 1 zu verhindern, indem das Paar erster Flächen 21 beispielsweise keilförmig ausgebildet ist, so dass eine Selbsthemmung in Richtung zu der Innenseite des Körperimplantats erzeugt wird. Im Gegensatz hierzu sind die zweiten Flächen 22 derart konfiguriert, um ein Verlagern des Markerelements 20 in einer Richtung zu der Aussenseite des Körperimplantats 1 zu verhindern, indem das Paar zweiter Flächen 22 beispielsweise keilförmig ausgebildet ist, so dass eine Selbsthemmung in Richtung zu der Aussenseite des Körperimplantats 1 erzeugt wird. Auf diese Weise wird das Markerelement 20 in dem Ausschnitt 10 fixiert.

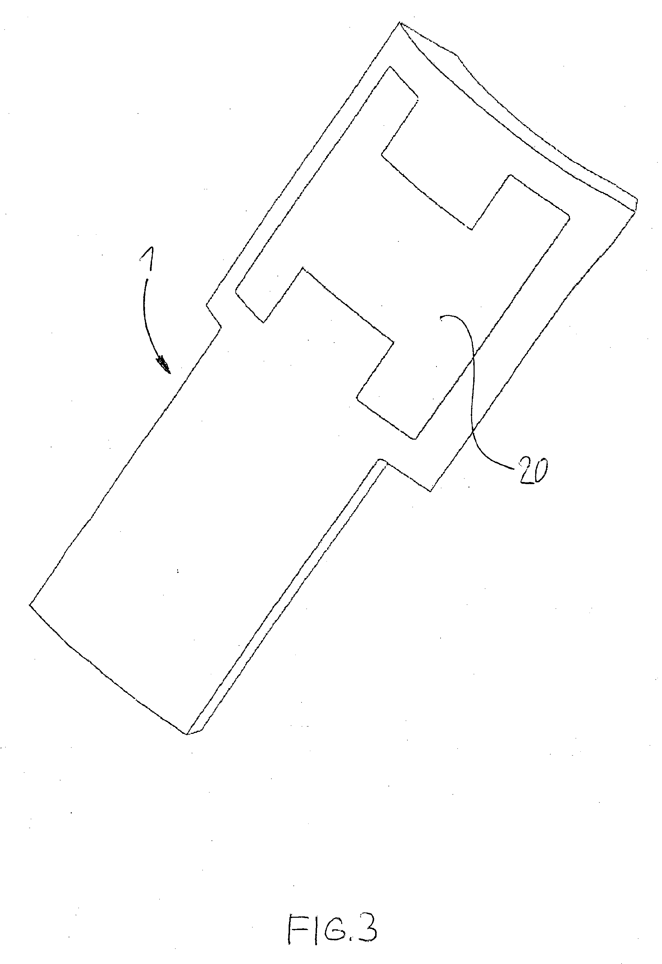

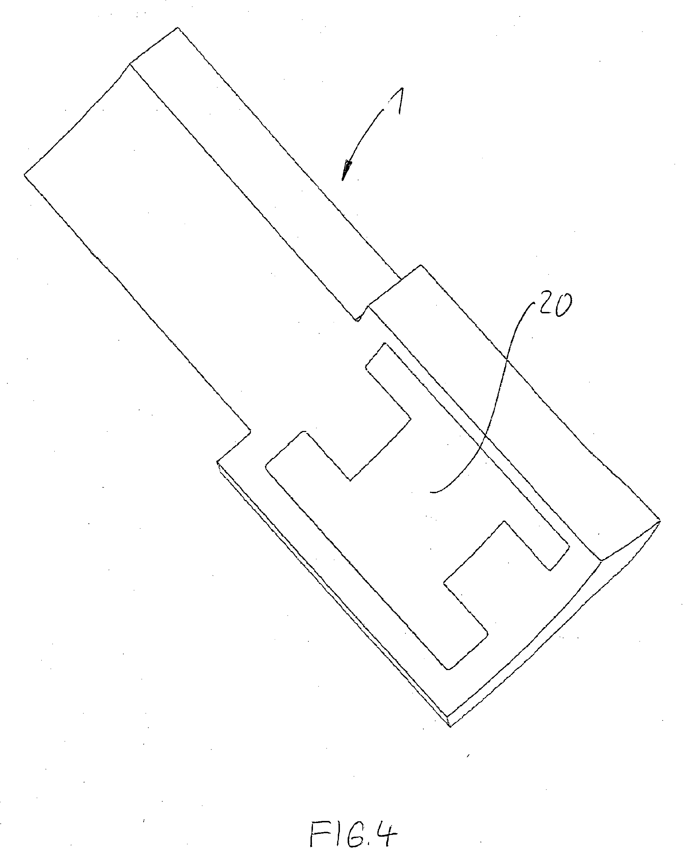

Wenn das Markerelement 20 in den Ausschnitt 10 eingesetzt ist, füllt es den Ausschnitt 10 bevorzugt im wesentlichen vollständig aus und bildet an der Innenseite I sowie der Außenseite A des Körperimplantats 1 eine im wesentlichen ebene Fläche mit den von dem Körperimplantat 1 angrenzenden bzw. benachbarten Flächen, wie insbesondere in den Figuren 3 und 4 gezeigt ist.

Vorzugsweise wird das Markerelement 20 in den Ausschnitt 10 eingesetzt, indem der Abschnitt des Körperimplantats 1 mit dem bogenförmigen Ausschnitt 10 im wesentlichen platt gedrückt wird, um auf elastisch rückstellfähige Weise eine im wesentlichen flache Gestalt anzunehmen. In dieser verformten Konfiguration des Ausschnitts 10 kann das Markerelement 20 eingesetzt werden. Nach dem Zurückkehren zu der ursprünglichen Bogenform wirkt die Hinterschneidung der ersten und zweiten Flächen 21, 22, um das Markerelement 20 zu fixieren. Derart wird das Markerelement 20 sozusagen in den Ausschnitt 10 eingeklipst. Das Markerelement 20 kann jedoch auch durch Vernieten oder Verpressen in dem Ausschnitt 10 fixiert werden.

Vorzugsweise sind die ersten und zweiten Flächen 21, 22 eben ausgebildet, um im wesentlichen eine Keilform zu bilden. Die Formgebung der Flächen 21, 22 ist jedoch nicht hierauf beschränkt. Es können genauso gekrümmte, gestufte, gezahnte Flächen etc. angewandt werden.

Das Markerelement 20 ist in dem eingeklipsten, eingepressten bzw. eingenieteten

Zustand, der in den Figs. 3 und 4 gezeigt ist, durch die Hinterschneidung des ersten Paars Flächen 11, 21 sowie die Hinterschneidung des zweiten Paars Flächen 12, 22 in dem Ausschnitt 10 fixiert. In anderen Worten bildet ein Paar Flächen einen in eine Richtung ausgerichteten Keil und das andere Paar Flächen einen Keil, der in die hierzu entgegengesetzte Richtung ausgerichtet ist. Anders ausgedrückt sind die Normalen auf die Ebenen des einen Paares gegenüber einer (hypothetischen) Umfangsrichtung X in einer Richtung geneigt, während die Normalen auf die Ebenen des anderen Paars gegenüber der Umfangsrichtung X entgegengesetzt geneigt sind. Das Markerelement 20 ist somit in dem eingenieteten oder eingepreßten Zustand durch Formschluß in dem Ausschnitt 10 fixiert. Somit ist eine Art Doppelkeil-Struktur erzielt, in der zwei entgegengesetzt orientierte Keile eine formschlüssige Verrastung des Markerelements 20 erzielen, so daß das Markerelement 20 weder nach Außen A noch nach Innen I aus dem Ausschnitt 10 entweichen bzw. aus dem Körperimplantat entfernt werden kann.

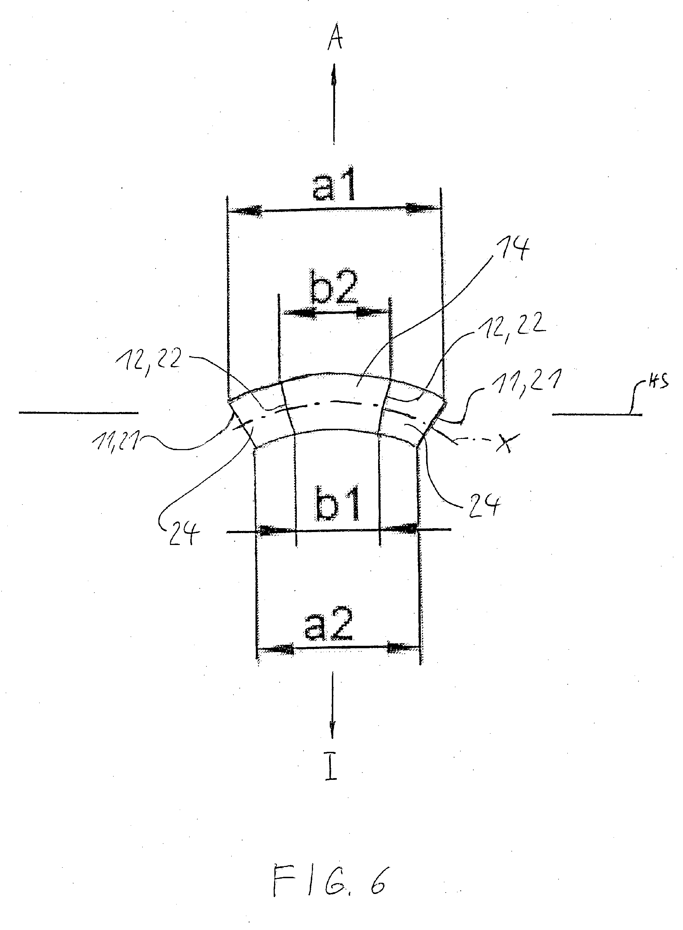

Dieser Formschluß bzw. die Keilbildung mittels der ersten und zweiten vorzugsweise im wesentlichen ebener Flächen 11, 12, 21, 22 soll mittels des Schnitts A - A von Fig. 5, der in Fig. 6 gezeigt ist, näher erläutert werden. Die ersten ebenen Flächen 11, 21 haben am Außenumfang des Körperimplantats 1 einen horizontalen Abstand a1 und am inneren Umfang des Körperimplantats 1 einen horizontalen Abstand a2. Die Abstände a1, a2 werden entlang einer Geraden HS gemessen, welche vorzugsweise im wesentlichen senkrecht auf die radiale Richtung des Stents und/oder parallel zur Tangenten an den Außenumfang des Körperimplantats 1 bzw. Stents in dem entsprechenden Bereich ist. Der horizontale Abstand a1 ist größer als der horizontale Abstand a2. Aufgrund dessen kann sich das Markerelement 20 nicht in Richtung I auf das Innere des Körperimplantats 1 bewegen, das heißt in Fig. 6 nach unten, weil die ersten ebenen Flächen 21 des Markerelements 20 sich mit den ersten ebenen Flächen 11 des Ausschnitts 10 einen Formschluß bilden, um eine Bewegung in Fig. 6 nach unten unmöglich zu machen.

Andererseits ist ein horizontaler Abstand b1 der zweiten ebenen Flächen 12, 22 am Innenumfang kleiner als ein horizontaler Abstand b2 am Außenumfang des Körperimplantats 1. Derart bilden die zweiten ebenen Flächen 12 des Ausschnitts 10 einen Keil, der eine Bewegung des Markerelements 20 in Richtung A nach außen, das heißt in der Richtung nach oben in Fig. 6 unmöglich macht. Aufgrund dieser Keilwirkung der ersten und zweiten ebenen Flächen 11, 12, 21, 22 ist das Markerelement 20 durch Formschluß in dem Ausschnitt 10 fixiert.

Vor dem Einsetzen des Markerelements 20 in den Ausschnitt 10 hat dieses im Falle des Verpressens eine geringfügig abweichende Form, so daß ein Einsetzen möglich ist. Hierzu reicht es aus, daß zumindest eine aus den ersten ebenen Flächen 21 und den zweiten ebenen Flächen 22 eine etwas abweichende Form hat, um ein Einsetzen zu ermöglichen. Beispielsweise kann das Markerelement 20 so gebildet sein, daß der Abstand b1 zwischen den zweiten ebenen Flächen 22 an dem Innenumfang im wesentlichen dem Abstand b2 entspricht. Derart könnte das Markerelement 20 in den Ausschnitt 10 eingesetzt werden. Danach erfolgt ein Einniet- bzw. Preßvorgang, der dazu führt, daß das Markerelement 20 sich in die endgültige Form im wesentlichen in Übereinstimmung mit der Form des Ausschnitts 10 verformt. Durch entsprechendes Fließen bzw. Verpressen des Materials kann sich der Abstand zwischen den zweiten ebenen Flächen 22, der ursprünglich im wesentlichen dem Abstand b2 entspricht, derart verringern, daß der Abstand zwischen den zweiten ebenen Flächen 22 am Innenumfang dem Abstand b1 entspricht.

Es versteht sich, daß die Erfindung nicht auf eine Veränderung des Abstands b2 auf den Abstand b1 beschränkt ist, sondern das Markerelement 20 auch von innen eingesetzt werden könnte, wenn die ersten ebenen Flächen 21 im ursprünglichen nicht eingesetzten Zustand des Markerelements 20 einen Abstand a2 aufweisen würden, um ein Einsetzen von der Innenseite I zu ermöglichen und danach durch Vernieten bzw. Verpressen den Abstand am Außenumfang der ersten ebenen Flächen 21 auf den horizontalen Abstand a1 zu bringen.

Vorzugsweise wird das Markerelement 20 jedoch von der Außenseite A in den Ausschnitt 10 eingesetzt, indem der Abstand b2 auf den Abstand b1 durch Einpressen bzw. Vernieten verringert wird.

In dem gezeigten Ausführungsbeispiel hat das Markerelement 20 im wesentlichen die Form eines Buchstaben H mit einem Mittelsteg 23 und einem Paar Längsstege 24. Die ersten und zweiten ebenen Flächen 21, 22 sind dabei an den Längsstegen 24 ausgebildet. Derart bildet das erste Paar ebener Flächen 21 an den Außenseiten der Längsstege 24 einen Keil zum Verhindern der Bewegung des Markerelements 20 in Richtung zu der Innenseite I des Körperimplantats 1, während das Paar zweiter ebener Flächen 22 an den Längsstegen 24 eine Bewegung des Markerelements 20 zu der Außenseite A des Körperimplantats 1 verhindert. Die ebenen Flächen 21, 22 müssen dabei nicht über die gesamte Länge der Längsstege 24 angeordnet werden.

Wie bereits angemerkt, ist die Erfindung nicht auf die Ausbildung des Markerelements 20 in der Form eines Buchstaben H beschränkt, sondern kann jede andere Form haben, die das Ausbilden von zwei entgegengesetzt gerichteten Keilen mit Hinterschneidungen in Richtung zu der Außenseite A des Körperimplantats 1 und einer weiteren Hinterschneidung in Richtung zu der Innenseite I des Körperimplantats 1 ermöglicht. Aufgrund der Keilwirkung der ersten und zweiten ebenen Flächen 21, 22 in Verbindung mit den ersten und zweiten ebenen Flächen 11, 12 des Ausschnitts 10 wird ein Formschluß zwischen Ausschnitt 10 und Markerelement 20 gebildet, um das Markerelement 20 durch entsprechendes Verformen bzw. Verpressen innerhalb des Ausschnitts 10 vollständig in dem Ausschnitt 10 zu fixieren.

Vorzugsweise ist eine Materialmenge des unverformten Markerelements 20 so bemessen, daß das Markerelement 20 nach dem Einpressen in den Ausschnitt 10 diesen im wesentlichen vollständig ausfüllt und an der Innen- und Außenseite I, A des Körperimplantats 1 mit der Wandung des Körperimplantats 1 im wesentlichen ebene Flächen bildet, um mit den angrenzenden Flächen im wesentlichen bündig abzuschließen.

Der Ausschnitt 10 kann somit sehr einfach durch Laserschneiden gebildet werden, indem einfach gerade Schnitte in dem Körperimplantat 1 geformt werden. Es ist somit nicht notwendig, komplizierte Formen, wie beispielsweise Konusformen und dergleichen, zum Bilden eines Formschlusses zu erzeugen. Derart kann der Ausschnitt 10 sehr einfach hergestellt werden.

Das Markerelement 20 wird vorzugsweise aus einem Rohr ausgeschnitten, um eine Kreisbogenform im wesentlichen gleich der Kreisbogenform des Körperimplantats 1 zu haben. Die Erfindung ist jedoch nicht hierauf beschränkt. Der Ausschnitt 10 kann auch in einem ebenen Querschnittsabschnitt eines Körperimplantats 1 gebildet werden und die entsprechenden ebenen Flächen zum Bilden eines Formschlusses aufweisen.

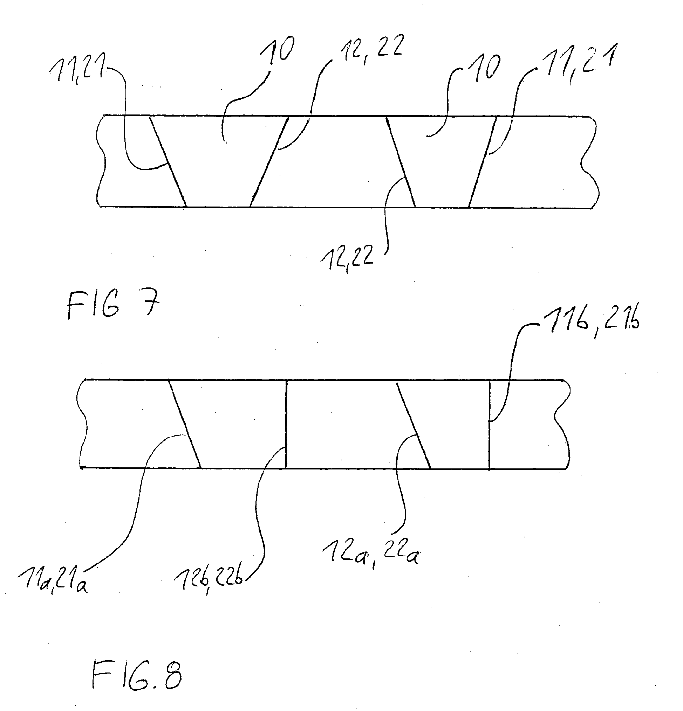

Wie des weiteren in Fig. 7 gezeigt ist, kann der Ausschnitt 10 auch in einem geradlinigen Abschnitt des Körperimplantats 1 angeordnet sein.

Darüber hinaus kann die Hinterschneidung auch so gebildet werden, dass nur eine ebene Fläche 11a, 21a, 12a 22a des Paars ebener Flächen hinterschnitten ist, während die andere ebene Fläche 11b, 21b, 12b, 22b des Paars ebener Flächen keine Hinterschneidung aufweist, indem sie im wesentlichen senkrecht zu der Körperimplantatoberfläche verläuft, das heißt mit der Oberfläche des Körperimplantats 1 einen rechten Winkel bildet.

Wie in dem Ausführungsbeispiel gezeigt ist, hat der Ausschnitt 10 ein Paar Vorsprünge 14, die die zweiten ebenen Flächen 12 aufweisen. Es versteht sich, daß auch nur ein Vorsprung 14 angeordnet werden kann.

Obwohl im Falle der Kreisbogenform des Ausschnitts 10 und des Markerelements 20 die ebenen Flächen im wesentlichen gegenüber der radialen Richtung des Körperimplantats 1 hinterschnitten bzw. geneigt sind, kann die Hinterschneidung auch bezüglich einer von der radialen Richtung abweichenden Richtung vorgesehen werden.

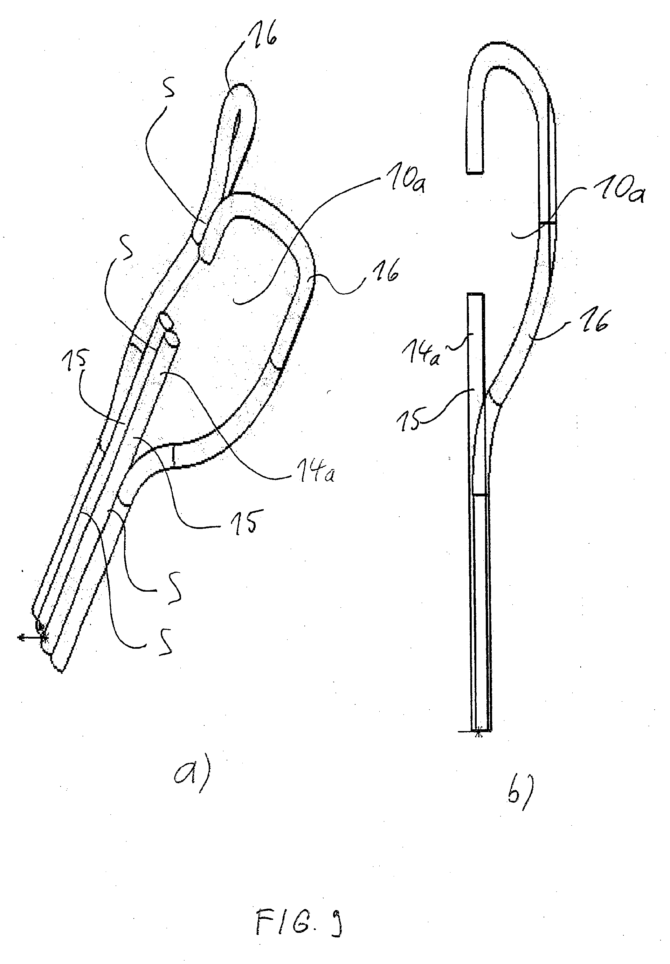

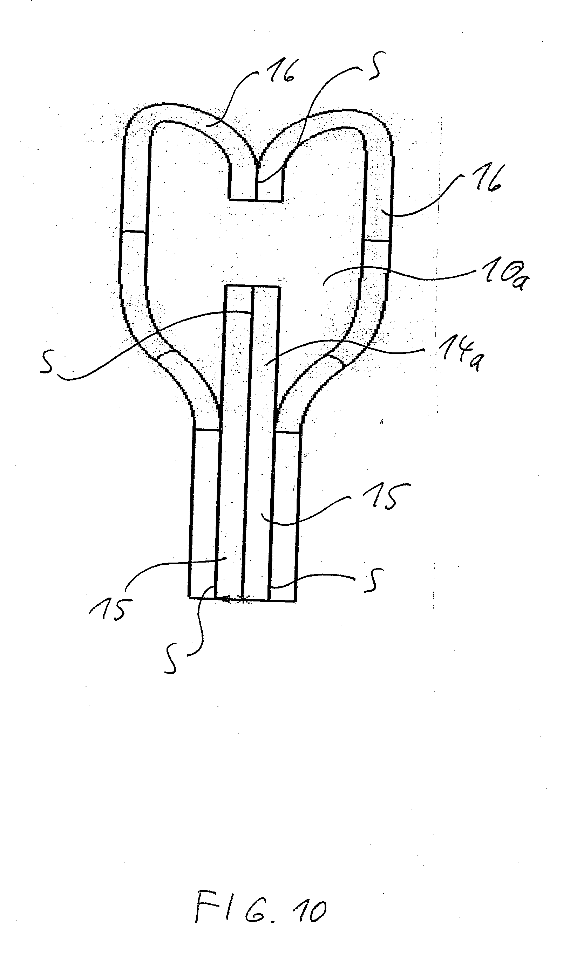

Fig. 9 und 10 zeigen einen Ausschnitt 10a eines zweiten Ausführungsbeispiels, der aus verschweißten Drähten 15, 16 zusammengesetzt ist. Dabei werden vorzugsweise zwei im wesentlichen gerade erste Drähte 15 an einer Vielzahl von Schweißpunkten S miteinander verschweißt. Des weiteren werden zwei weitere zweite Drähte 16 an das Paar erster Drähte 15 angeschweißt. Die zweiten Drähte 16 werden schleifenförmig gebogen und die ersten Drähte 15 stehen in die durch die zweiten Drähte 16 gebildete Schleife hinein vor, um einen Vorsprung 14a zu bilden.

Auf diese Weise kann ein dreidimensionaler Ausschnitt 10a in der Gestalt eines Buchstaben H wie im ersten Ausführungsbeispiel gebildet werden. Vorzugsweise hat das (nicht gezeigte) Markerelement im zweiten Ausführungsbeispiel konkav abgerundete Kanten, um einen formschlüssigen Eingriff mit den Drähten 15, 16 zu ermöglichen. Durch Plattdrücken des dreidimensionalen geschweißten Aussschnitts 10a kann das Markerelement eingesetzt werden. Wenn die Kraft zum Plattdücken des Ausschnitts 10a weggenommen wird, stellt sich das Gebilde aus den Drähten 15, 16 auf elastische Weise zurück, um seine ursprüngliche Form anzunehmen.

In dieser ursprünglichen dreidimensionalen Form des Ausschnitts 10a ist das Markerelement formschlüssig fixiert, d.h. durch Selbsthemmung in dem Ausschnitt 10a befestigt.

### Bezugszeichenliste

- 1: Körperimplantat
- 10, 10a: Ausschnitt
- 11: erste Fläche
- 11a: erste Fläche mit Hinterschneidung
- 11b: erste Fläche ohne Hinterschneidung
- 12: zweite Fläche
- 12a: zweite Fläche mit Hinterschneidung
- 12b: zweite Fläche ohne Hinterschneidung
- 14, 14a: Vorsprung
- 15: erster Draht
- 16: zweiter Draht
- 20: Markerelement
- 21: erste Fläche
- 21a: erste Fläche mit Hinterschneidung
- 21b: erste Fläche ohne Hinterschneidung
- 22: zweite Fläche
- 22a: zweite Fläche mit Hinterschneidung
- 22b: zweite Fläche ohne Hinterschneidung
- 23: Mittelsteg
- 24: Längssteg
- A: Außenseite
- I: Innenseite

## Patentansprüche

1. Körperimplantat (1), insbesondere Stent, zum Einführen oder Implantieren in einen lebenden Körper mit einem Markerelement (20) aus einem röntgensichtbaren Material, das in einen Ausschnitt (10; 10a) des Körperimplantats (1) eingesetzt und dort fixiert ist, wobei der Ausschnitt (10) zwei Paare Flächen (11, 12) aufweist, die so konfiguriert sind, dass ein Paar erster Flächen (11) eine erste Hinterschneidung in einer Richtung zu dem Inneren (I) des Körperimplantats (1) aufweist, während das andere Paar zweiter Flächen (12) eine zweite Hinterschneidung in der im wesentlichen entgegengesetzten Richtung (A) aufweist.

2. Körperimplantat (1) nach Anspruch 1, wobei das Markerelement (20) komplementäre Paare Flächen (21, 22) aufweist und eines der Paare Flächen (21) im wesentlichen einen Keil an dem Markerelement (20) bildet und das andere Paar Flächen (12) im wesentlichen einen Keil an dem Körperimplantat (1) bildet.

3. Körperimplantat (1) nach einem der vorherigen Ansprüche, wobei der Ausschnitt (10) in einem zumindest teilweise runden oder bogenförmigen Abschnitt des Körperimplantats (1) angeordnet ist und die Flächen (11, 12) in Umfangsrichtung des Körperimplantats (1) versetzt sind und/oder das Markerelement (20) einen Querschnitt in der Form eines Kreisbogens hat.

4. Körperimplantat (1) nach einem der vorherigen Ansprüche, wobei das Markerelement (20) in den Ausschnitt (10) eingeklipst, eingepresst oder eingenietet ist und/oder der Ausschnitt (10) im wesentlichen die Form eines Buchstaben A, H, N, V oder Z hat.

5. Körperimplantat (1) nach einem der vorherigen Ansprüche, wobei das Markerelement (20) nach dem Einklipsen, Einpressen bzw. Einnieten innen und/oder außen im wesentlichen eben mit einer Implantatstruktur des Körperimplantats (1) ist.

6. Körperimplantat (1) nach einem der vorherigen Ansprüche, wobei das Markerelement (2) ein Material mit hoher Absorption für Röntgenstrahlen wie beispielsweise Gold, Platin, Tantal, Platinlegierung, Platin-Iridium, Niob aufweist.

7. Verfahren zum Verbessern der Röntgensichtbarkeit eines Körperimplantats (1), insbesondere eines Stents, mit den Schritten:
Formen eines Ausschnitts (10) in dem Körperimplantat (1) mittels eines Lasers, so dass der Ausschnitt (10) zwei Paare Flächen (11, 12) aufweist und ein Paar Flächen (11) eine erste Hinterschneidung in einer Richtung zu dem Inneren (I) des Körperimplantats (1) aufweist, während das andere Paar Flächen (12) eine zweite Hinterschneidung in der im wesentlichen entgegengesetzten Richtung aufweist, und
Einsetzen und Fixieren eines Markerelements (20) aus einem Material mit hoher Absorption für Röntgenstrahlen in dem Ausschnitt (10).

8. Verfahren nach Anspruch 7, wobei das Markerelement (20) vor dem Einsetzen in den Ausschnitt (10) eine im wesentlichen komplementäre Form bezüglich der Form des Ausschnitts (10) hat, jedoch höchstens eine der beiden Hinterschneidungen aufweist, und wobei das Markerelement (20) nach dem Einsetzen so verformt wird, dass es beide Hinterschneidungen aufweist.

9. Verfahren nach Anspruch 7 oder 8, wobei der Ausschnitt (10) in einem zumindest teilweise runden oder bogenförmigen Abschnitt des Körperimplantats (1) geformt wird und die Flächen in Umfangsrichtung des Körperimplantats (1) versetzt geformt werden und/oder das Markerelement (20) aus einem Rohr geschnitten wird, um einen Querschnitt in der Form eines Kreisbogens zu haben.

10. Verfahren zum Herstellen eines Körperimplantats (1), insbesondere eines Stents, mit einem röntgensichtbaren Markerelement (20) mit den Schritten:
Formen eines Ausschnitts (10) in dem Körperimplantat (1), so dass der Ausschnitt (10) zwei Paare Flächen aufweist und ein Paar Flächen eine erste Hinterschneidung in einer Richtung zu dem Inneren des Körperimplantats (1) aufweist, während das andere Paar Flächen eine zweite Hinterschneidung in der im wesentlichen entgegengesetzten Richtung aufweist, und
Einpressen bzw. Einnieten eines Markerelements (20) aus einem Material mit hoher Absorption für Röntgenstrahlen.
